# EUROPEAN PATENT APPLICATION

(11) **EP 1 591 788 A1**
(43) Date of publication of application: **02.11.2005**
(21) Application number: 05103383.5
(22) Date of filing: 26.04.2005
(51) Int. Cl.: G01N 33/32, C09D 11/00, G01N 35/00

(54) **Method to monitor the quality of dispersion formulations**

(30) Priority: 27.04.2004 EP 04101757
(71) Applicant: AGFA-GEVAERT, 2640 Mortsel (BE)
(72) Inventor: Desie, Guido, 2640, Mortsel (BE); Vanmaele, Luc, 2640, Mortsel (BE); Deroover, Geert, B2640, Mortsel (BE)

(57) **Abstract**

In a combinatorial method of making and testing an array of nanoparticle formulations comprising the steps of
- making multi-compositional formulations;
- improving the dispersion quality of these formulations by high speed parallel homogenizing;
- rapid serial, semi-parallel or parallel characterising said formulations, said step of high speed parallel homogenizing proceeds by providing energy to said dispersion via an array of tips, wherein said energy is ultrasonic energy or high shear mixing energy.

## Description

### FIELD OF THE INVENTION

The present invention is related to a workflow for high speed material design, more particularly to the development of ink-jet inks in an array of nanoparticular dispersion formulations, and to a workflow for making, improving and screening or characterising said array in order to control critical properties thereof.

### BACKGROUND OF THE INVENTION

As disclosed in EP-A 0 878 516 pigment based inks are typically prepared by dispersing pigment agglomerates, formed when individual pigment particles cluster together in a dispersant. The size of the pigment agglomerates may be reduced by grinding the pigment dispersion using conventional grinding media such as glass, stainless steel, or zirconium oxide. Conventional grinding media however have been found to be unacceptable because they either increase the pH of the dispersion to an unacceptable level, resulting in inks having incompatibility with ink processing and printing equipment or result in contamination of the dispersion, thereby leading to discolouration of inks prepared from the above-described pigment dispersions. A solution therefore is based on the discovery that plastic media for grinding colourant dispersions, while satisfactorily reducing the colourant agglomerate size in the pigment dispersion, do not unacceptably alter the pH or unduly contaminate the dispersion, moreover unexpectedly resulting in an ink composition having a superior colour gamut.

In WO 02/14377 a method has been described to develop nanodispersions in a combinatorial way as, e.g., by means of parallel precipitation technology. Therein it has been described that standard grinding processes starting from solid bulk materials do not typically result in particles with average diameters of less than 0.5 µm. Particle size and distribution depend on a variety of parameters like the type of mill or the crushing parts (e.g. silica) and the need to remove the crushing parts after milling, that leads to further problems related with heterogeneous systems. Larger particle sizes of milled materials indeed require additives to stabilise a dispersion thereof in order to avoid agglomeration, flocculation, sedimentation and flotation. An alternative to start from the molecular solution and to form particles by precipitation results in problems related to crystal growth by Ostwald ripening and/or particle agglomeration, further resulting in sedimentation and/or flotation. The synthesis of dispersion formulations in physically separate synthesis regions proceeds in situ, as described in that application. As is taught therein mild agitition during that synthesis was provided by repeatedly aspirating and dispensing fluid from the vials, and an additional low energy agitation was provided by making use of a small, bench-top ultrasonic mixer that was also used in order to agitate the stock solutions before dispensing said solutions into glass vials, wherein the synthesis should proceed. Said dispersion synthesis method however provides no information about dispersion quality factors obtained thereby. In US-A 6,508,104 it has been established to add ultrasonic energy by sonicating a liquid suspension of first particles and analyzing the thereby separated second particles, in order to determine adhesion force relationships between both of them. In fact the said US-A 6,508,104 discloses a separation technique, followed by a quantitative analysis procedure.

From the TOMTEC® Autogizer leaflet it has been learnt that automation for all homogenising tasks (as e.g. evaluation of brain uptake of drug candidates in drug discovery) is attainable by means of a device, developed to homogenise cell-cultures via high speed homogenisers, installed in a parallel frame. Processing of brain samples thus proceeds at least 10 times faster than manual homogenization and makes use of much less amounts of water and solvents, in favour of the environment.

With respect to mechanical pulverising dispersion procedures until the average particle size of pulverise-dispersed agglomerate pigment particles reaches sizes in the range from 10 to 300 nm, in favour of colour density in ink-jet recording materials, it is taught in US-A's 5,958,168 and 6,270,837 to apply a mechanical pulverizing procedure under a high shearing force, wherein as a breaking down method of agglomerate pigment particles in the form of lumps up to finely divided material, mechanical pulverizing means including ultrasonic pulverisers, high speed rotation mills, roll mills, container-drived medium mills, medium stirring mills, jet mills, mortars, sand grinders, pressure-type homogenisers and Cowless dispersers are advantageously applied.

Addition of ultrasonic energy to pigmented inks has further been described in WO 98/44058, wherein the mixture of pigment, polymeric dispersant and aqueous medium is subjected to ultrasonic energy, whereby a narrower particle size distribution is obtained in the ink and wherein, in a preferred embodiment, the step of subjecting the mixture to ultrasonic energy is used in combination with a dispersion step, selected from the group consisting of media milling, two-roll milling and microfluidizing.

Furtheron as described in US-A 6,136,890 dispersants are typically employed in pigmented ink jet inks to stabilise the dispersion to prevent agglomeration or flocculation of the pigment particles and consequent settling of the pigment particles out of the dispersion. It is theorised that dispersing compounds behave by adsorbing onto a pigment surface to create a protective layer around each pigment particle to counteract attractive forces between the particles, thereby preventing agglomeration and flocculation. However there is a need for dispersants useful in ink jet inks that exhibit one or more of the above-described properties, including: the ability to disperse a pigment into a stable dispersion that will not suffer from agglomeration, flocculation, or kogation. A need simultaneously exists for ink jet printer inks that are free or substantially free of organic solvents.

Pigmented ink comprising a liquid medium and a pigment dispersed within the liquid medium, wherein the pigment is stabilised within the liquid medium by a polyurethane dispersant and wherein the polyurethane dispersant comprises a dispersant group, therefor brings a solution.

In US-A 6,245,832 it is described that for dispersing the pigment, a dispersing apparatus using no dispersion media such as glass, ceramic or metal balls or beads is preferred. If a dispersing apparatus using a dispersion media is used, the dispersion media or dispersion vessel is abraded during the grinding or dispersion of the pigment and a large amount of inorganic impurities are often mixed into the pigment dispersion solution or ink. When a dispersing apparatus using a dispersion media is used, it is preferred, if desired, to remove inorganic impurities mixed into the dispersion solution or ink. As a dispersing apparatus using no dispersion media, an ultrasonic homogeniser and a highpressure homogeniser are particularly preferred. In the case of using an ultrasonic homogeniser, the pigment is preferably dispersed after it is defoamed or deaerated by vacuum evacuation or heating or by means of a commercially available defoaming/deaerating apparatus. In US-A 6,478,862 it has been described that the pigment ink has a nominal particle size of less than about 0.2 µm; and is subjected to ultrasonic mixing using ultrasonic energy.

A method for regulating the average particle diameter of a pigment for an ink composition possessing excellent anti-clogging properties and ejection stability, has been described in US-A 6,562,117 wherein said method comprises the steps of dispersing the pigment in a solvent to prepare a stock pigment dispersion and subjecting the stock pigment dispersion to crossflow membrane filtration.

From our own experience, when making inks having the final composition as envisaged, those inks comprise particle agglomerates in a medium of polymeric stabilisers and additives, but said inks are unacceptable for practical use, due to coarse agglomerates. The "dispersion process" normally involves addition of e.g. zirconium oxide pearls and an overnight roller bench step, but this treatment step imposes considerable drawbacks as explained earlier. When the pearls have to be removed the next day and the resulting dispersion is adjusted to fit into the screening process, this process moreover involves a dilution step for spectral- or Particle-Size-Distribution (PSD)-analysis.

Whereas in all of those applications serial dispersion preparation methods have been described, it is clear that these processes, predominantly based on application of shear forces, are time-consuming, in part due to the large scale on which the experiments are performed, and that high speed preparation of well-dispersed complex dispersions is not easy: normally the composition is prepared, followed by a sequential "dispersing" step so that the speed of material design is very slow.

It is clear that there is a stringent demand for an alternative and quick method, preferably on smaller scale, so that the full workflow can be automated, without intermediate bottlenecks limiting the overall speed, in order to be able to quickly design improved ink-jet ink dispersions.

### OBJECTS AND SUMMARY OF THE INVENTION

Therefore it is an object of the present invention to develop fine well-dispersed formulations. More particularly it is an object of the present invention to prepare fine well-dispersed nanoparticle formulations of pigment based ink jet inks, starting from agglomerates of pigments with unacceptable large sizes.

It is further an ever lasting object to develop a faster method than hitherto available, preferably on smaller scales than ever applied, in order to consume smaller amounts of material, not only without reducing the number of said experiments, but opposite thereto even increase said number of experiments in order to improve research efforts and to accelerate research: as more formulations are envisaged to be prepared and screened in less time on a smaller scale, it is a further object to decrease the cost per sample to a large extent.

Further objects will become apparent from the description hereinafter.

A solution for the problem set forth hereinbefore has been realised by a method having the specific features set out in claim 1. Specific features for preferred embodiments of the invention are set out in the dependent claims.

Further advantages and embodiments of the present invention will become apparent from the following description [and drawings].

### BRIEF DESCRIPTION OF THE DRAWINGS

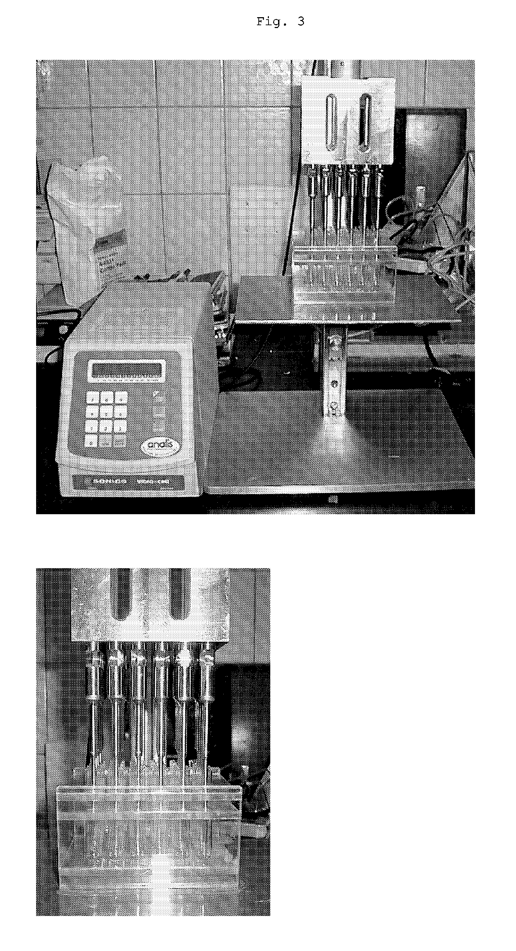

Taking into account the definitions of
M, representing the number of samples or elements in an array (as e.g. in an I x J - array);
N, representing the number of ultrasonic heads, probes or tips in an array (as e.g. in a K x L - array) and
X, representing the number of operations (and equal to M/N as preferred above exceeding said ratio M/N as in Fig. 2):

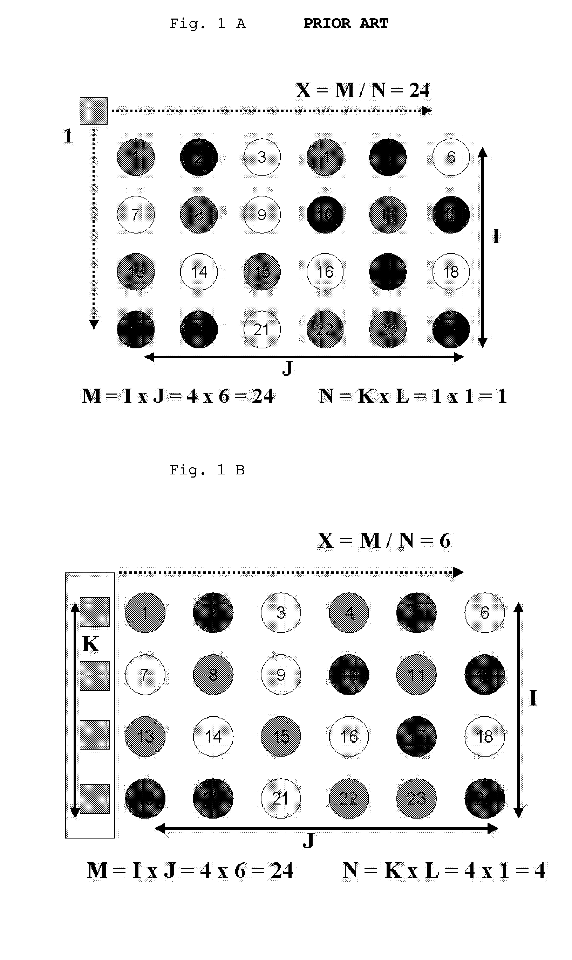

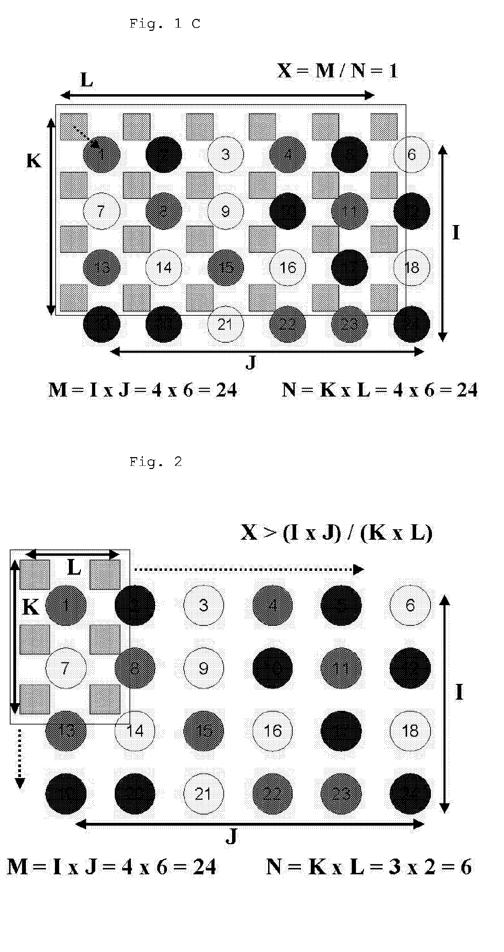

Fig. 1 shows examples of standard serial experimentation methodologies (A), rapid serial/parallel experimentation methodologies (B) and full parallel experimentation methodologies (C).
   A. Prior art: dispersion method in one-by-one steps (N = K x L = 1, M = I x J = 24; X = M/N = 24).
      The array of samples to be treated in this example is I (4) x J (6) = 24. The head of the treating device has a single head probe, also called "tip": so to do the full 24 dispersing steps 24 serial operations have to be done (X = M / N = 24 operations)
   B. Linear arrangement (example: N = K x L = 4 x 1 = 4, M = I x J = 24; X = M/N = 6).
      The array of samples to be treated in this example is I (4) x J (6) = 24. The head of the treating device has a N(4)-head probe (N(4) tips): so to do the full 24 dispersing steps 6 serial/parallel operations have to be done using the 4-probe head in a rapid serial/parallel action (X = M / N = 24 / 4 = 6 operations)
   C. Two-dimensional arrangement (example: N = K x L = 4 x 6 = 24, M = I x J =24; X = M/N = 1).
      The array of samples to be treated in this example is I (4) x J (6) = 24. The head of the treating device has a K x L (24)-head probe (K x L (24) tips)): so to do the full 24 dispersing steps a single parallel operation has to be done using the 24-probe head in a rapid parallel action (X = M / N = 24 / 24 = 1 operation).
Fig. 2 shows the ability to prepare and test I x J arrangements in a fast serial parallel setup with K x L probe heads or tips. In this picture an example is shown to treat an I x J matrix of dispersions with a multi-probe head having a K x L matrix of dispersing tips. It is clear from this figure that in the most ideal situation only (I x J)/(K x L) operations are required to treat all samples. With the example as illustrated in this figure it is clear that 6 operations are required as depicted (3 translations in the J direction, combined with 2 translations in the I direction): not all probe tips have to be active all the time since there is no optimal suit with respect to the sample matrix. If the head is rotated by 90 degrees e.g. then a 6 x 4 sample matrix can be treated more efficiently with a 3 x 2 multi-tip probe, since now 2 translations are sufficient in the I direction, and also 2 translations are required in the J direction: ALL 24 samples can in this way be treated in 4 (2 x 2) operations while ALL probe tips are continuously active.
Fig. 3 shows a picture of the apparatus having "ultrasonic tips" for use in combinatorial dispersion preparation.

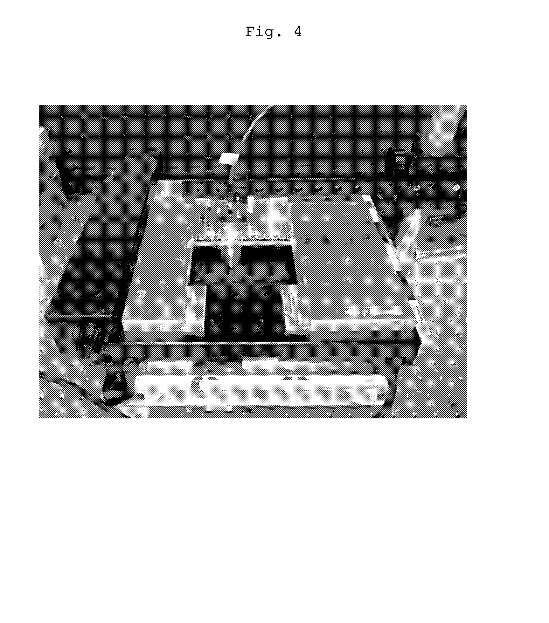

Fig. 4 shows a photograph of a custom made microtiter plate reader, offering the ability to determine a full visual spectrum in a fast serial way via a fiber-optic coupled oceanOptics® spectrum analyser being connected to a moving head on a XY-stage and connected to the spectrum analyser via an optical fiber, said device having both transmission and reflection measuring capabilities.

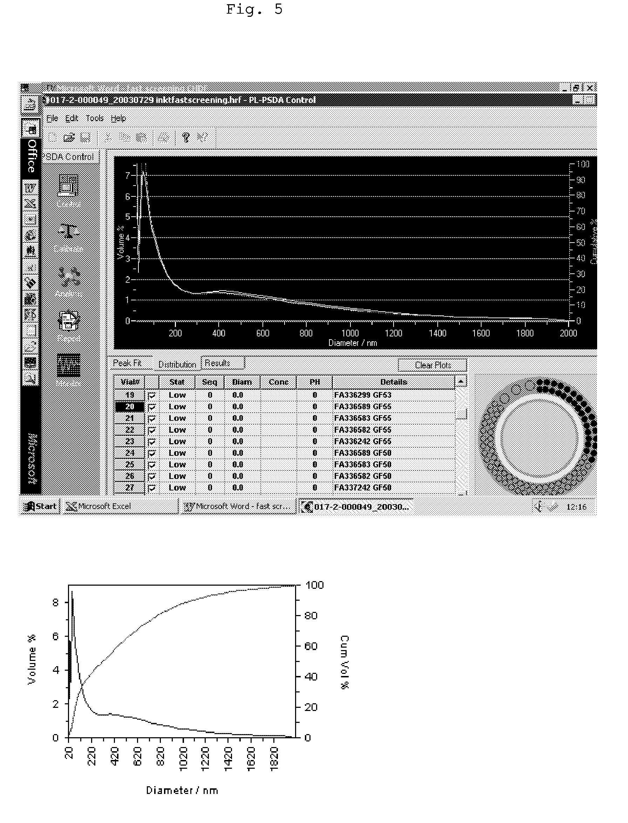

Fig. 5 shows an example of a PSD measurement performed on the automated PL-PSDA device, wherein the particle size distribution for a single ink is depicted. All samples are available in a caroussel so that fast serial measurements can proceed autonomously.

### DETAILED DESCRIPTION OF THE INVENTION

In the further description it is understood that the term "nanoparticle" refers to a particle having a size lower than 1 µm, and more preferably lower than 250 nm, i.e. more preferably from 10 nm to 100 nm and most preferably in the range between 30 and 100 nm. The term "nanoparticle formulations" refers to a composition wherein additives like viscosity modifying agents (thinners or thickeners), antioxidants, biocides, fungicides, pesticides, stabilizers and, optionally, one or more solvents, are present besides the main substance that should be dispersed to a "nanoparticle" size as defined hereinbefore.

According to the present invention a method has been provided to screen a lot of nanoparticle compositions, in particular for pigment based ink jet inks, with only minor consumption of consumables. It should be clear that no chemical synthesis is performed during the steps as described, but that a physico-chemical treatment step provides the improvement as envisaged. Furtheron it should also be clear that no analysis of separated phases while screening those nanoparticle compositions during the step of high speed parallel homogenizing said composition is performed. This method involves, in a first step, the addition of all of the components into the target receptable such as e.g. an EPA-vial, a small glass tube, or a microtiter plate. Powder and liquid handlers are used for the preparation of the raw pigment material being weighted by making use of gravimetric control on a powder handler such as e.g. an Autodose Powdernium® MTM robot, the diluted stock solutions of the other compositions and the solvents being dispensed by a liquid handler such as e.g. a Gilson XL222 robot. Furtheron the array of the tubes containing the different dispersions is arranged on an additional dispersion improving device such as e.g. a SONICS® 24-probe for ultrasonic excitation, a TOMTEC® Autogizer robot, or other devices. At the end of this dispersion improving step the resulting tubes are checked for dispersion quality characterisation by making use of screening methods.

Particularly suitable is the new product from SONICS® which offers ultrasonic probes for processing in microtiterplates.

All samples are treated simultaneously in such an ultrasonic excitation step for a time of a few hours. The 24-tips are automatically rinsed during a cleaning procedure. The vials are automatically diluted (e.g. in a microtiterplate for spectral analysis) or by making use of microtiterfilterplates in PSD-vials (for automatic PSD measurement).

Alternatively, the EPA-vials are used on a Tomtec Autogizer equipped with 5 parallel ultrasonic probes or high speed cutters as e.g. ultraturrax probes in a fast serial mode, to improve the dispersion quality during one more hour.

A 3-step cleaning process is further applied in order to prevent mixing of target solutions. This is thus a combined serial/parallel workflow, again followed by automated dilutions for automated screening.

Unlike ultrasonic baths or microplate horns that act as ultrasonic baths, the probes deliver the intensity directly into the sample, not through the vials where a great percentage of energy can be absorbed. Unlike single probes with grooved protrusions, these composite probes act as mechanical amplifiers. With ultrasonics the greater mass ratio between the probe upper section and the lower section, the greater the intensity at the probe tip. The SONICS® ultrasonic probes e.g. are 20 kHz multiple probes consisting of a coupler and multiple, replaceable stepped 3 mm microtips. Resonant frequency and length have been tested in order to ascertain that the excursion at the tip of each element is within specified tolerance. The 20 kHz 24-element probe from SONICS® consists of an aluminum coupler and a 24 stepped microtips (3 mm) for addition of ultrasonic energy in volumes of from 250 µl up to 10 ml.

According to the present invention a combinatorial method of making and testing an array of nanoparticle formulations is disclosed, said method comprising the steps of
- making multi-compositional formulations;
- improving the dispersion quality of these formulations by high speed parallel homogenizing;
- rapid serial, semi-parallel or parallel characterising said formulations,
characterised in that said step of high speed parallel homogenizing proceeds by providing energy to said dispersion via an array of tips.

According to the method of the present invention, said energy is ultrasonic energy and further according to the method of the present invention, in said step of high speed parallel homogenizing an N-tip probe is connected to an ultrasonic source, N being an integer.

It should be stressed and repeated that unlike ultrasonic baths or microplate horns that act as ultrasonic baths, the probes deliver the ultrasonic energy directly into the sample and that the said ultrasonic energy becomes provided by an N-tip ultrasonic probe to each tip of said N-tip ultrasonic probe, each tip acting thereby as an individual mechanical amplifier.

Apart therefrom it is understood that physico-chemically stable formulations improved and characterised by the combinatorial method of the present invention are not the result, and more particularly not during said step of high speed parallel homogenizing, of a method of combinatorial synthesis, starting from chemically reacting components required to obtain a desired composition as a result of a chemical reaction, or a combinatorial analysis of multi-compositional compositions, resulting from chemical reactions or physical interactions. Physico-chemically stable formulations, improved in dispersion quality by high speed (simultaneous) parallel homogenizing, further rapid serial, semi-parallel or parallel characterised by screening of said M formulations, should advantageously be uniform or homogeneous.

According to the method of the present invention, said N-tip ultrasonic probe is provided with N tips, arranged in a linear or in a two-dimensional arrangement.

According to the method of the present invention, said N-tip probe is provided with N tips, arranged in a linear or in a two-dimensional arrangement, in order to make M nanoparticle formulations, wherein N and M are integers.

Further according to the method of the present invention, N and M are related by the formula 4 ≤ N ≤ M, and it is most preferred according to the present invention that N and M are related by the formula M/N = n, wherein n is an integer, as integers provide an optimised utilisation and performance of this combinatorial method, leaving no site free. So according to a preferred embodiment of the method of the present invention, said N-tip ultrasonic probe is provided with N tips, arranged in a two-dimensional arrangement, wherein N and M are integers, related by the formula M/N = n, wherein n is an integer, thus having a value of at least 1.

Opposite to working in a serial mode, which still takes quite a lot of time, use of simultaneous parallel testing remarkably saves time as envisaged. This is not only the case when a linear array of N tips is used, N being 4 or more, wherein parallel steps from one linear array to another parallel array are performed, but even more when in a two-dimensional arrangement M tips are provided, preferably so that M is a multiple of N.

According to anoher embodiment in the method of the present invention, said energy is high shear mixing energy. Preferably said high shear mixing energy is provided via a high speed mixer with a rotation speed of more than 10,000 r.p.m.. Without being limitative superior magnetic driveless technology may be applied to dispersing volumes from 0.8 ml to 10 ml for viscosity ranges of the formulations from 1 to 5,000 mPas. Rotations in the range up to 30,000; more preferably in the range from 10,000 to 90,000 r.p.m., as e.g. about 75,000 r.p.m. can e.g. be provided by making use of the SilentCrusher® from Heidolph GmbH & Co, Schwabach, Germany.

According to the method of the present invention, said formulations are differing in at least one characteristic parameter, wherein said parameter is selected from the group consisting of composition, temperature and pH-value.

According to the method of the present invention, said compositions are differing in presence or absence of additives, and, in case of presence thereof, in concentrations of said additives. Preferably said additives are selected from the group consisting of viscosity modifying agents, i.e. thinners or thickeners, antioxidants, biocides, fungicides, pesticides, stabilisers and solvents. One or more solvents may optionally be present.

According to the method of the present invention, nanoparticle formulations selected from the group consisting of aqueous, solvent based, oil based and radiation curable ink-jet ink dispersion formulations, comprising at least a pigment and a binder, are advantageously prepared and tested as envisaged in the present invention.

The combinatorial method of making and testing an array of nanoparticle formulations is advantageously applied to ink-jet ink formulations, and is more particularly suitable for
- water based formulations, wherein the drying mechanism involves absorption, penetration and evaporation;
- oil based formulations, wherein the drying involves absorption and penetration;
- solvent based formulations, the drying of which primarily involves evaporation;
- hot melt or phase change formulations, in which the ink is liquid at the ejection temperature but solid at room temperature and wherein drying is replaced by solidification; and
- UV-curable ink formulations, in which drying is replaced by polymerization, and whereupon the rapid combinatorial method of making and testing an array of nanoparticle formulations, is advantageously applied.

According to the method of the present invention, said step of making multi-compositional formulations proceeds by addition of components in physically separate regions, said regions, situated on a substrate, being wells on a microtiterplate reactor or vials on a rack.

Furtheron according to the method of the present invention, said components are preferably delivered to said regions on said substrate by means of a robot comprising liquid and/or solid dispensing units.

In a preferred embodiment according to the method of the present invention, said step of improving the dispersion quality of these formulations proceeds by making use of X sequential actions of the said N-tip ultrasonic probe, M being X times N.

In a most preferred embodiment thereof M equals N, so that only 1 operation is required as illustrated e.g. in Fig. 1C.

Moreover according to another embodiment of the method of the present invention, said step of improving the dispersion quality of these formulations includes a pretreatment step. With respect thereto, according to the method of the present invention, said pretreatment step proceeds ultrasonically at a lower energy input than while performing said step of improving the dispersion quality. As ultrasonic energy input easily provokes temperature increase, magnetic stirring and shaking are recommended as alternative techniques. In another embodiment said pretreatment step thus proceeds by low shear mixing.

In addition according to the method of the present invention, said method further comprises a cleaning step.

The step of characterisation proceeds by screening of the improved formulations and is performed, according to the method of the present invention, by techniques selected from the group consisting of optical, electrical, magnetic, chemical, biochemical and physicochemical techniques. More particularly according to the present invention, the step of characterisation proceeds by screening of the improved formulations and is performed by parallel measurement techniques selected from the group consisting of optical, electrical, magnetic, chemical, biochemical and physicochemical techniques.

Measuring techniques are selected from the group consisting of Coulter counter analysis, light-scattering measurement, scanning Raman spectroscopy, scanning NMR spectroscopy, scanning probe spectroscopy including surface potentiometry, tunnelling current, atomic force, acoustic microscopy, shearing-stress microscopy, ultra fast photo excitation, electrostatic force microscope, tunneling induced photo emission microscope, magnetic force microscope, microwave field-induced surface harmonic generation microscope, nonlinear alternating-current tunnelling microscopy, near-field scanning optical microscopy, inelastic electron tunneling spectrometry, optical microscopy at different wavelengths; physicochemical (density, viscosity...), scanning optical ellipsometry for measuring dielectric constant and multilayer film thickness; scanning Eddy-current microscope; electron (diffraction) microscopy and spectrophotometry.

More preferably according to the method of the present invention the step of characterisation proceeds by screening of the improved formulations and is performed by an optical method selected from the group consisting of visual inspection of nanoparticle dispersion formulations, optical transmission at selected wavelength(s) and quasi-elastic light scattering.

With respect to the said temperature as a characteristic parameter, temperature reduction with at least 5°C is advantageously applied during the step of improving the dispersion quality, in the method according to the present invention.

In a particular embodiment according to the method of the present invention, a step of adding beads to the dispersion is included.

According to a preferred embodiment of the method of the present invention adding beads to the dispersion comprising a pigment is performed in a bead weight amount being larger than pigment weight amount. In a preferred embodiment according to the method of the present invention adding beads is performed in an amount of more than twice the said pigment weight amount. Further in a preferred embodiment according to the present invention said beads are composed of glass. Preferably said beads have a particle size diameter in the range from 25 µm to 200 µm.

According to the present invention said method is performed with a sample volume of less than 10 ml for each of said formulations, more preferably less than 5 ml and even most preferably less than 3 ml.

In the method according to the present invention said characterisation of formulations advantageously proceeds by directly screening of said formulations in a microtiter plate by means of spectral analysis. In a particular embodiment according to the present invention said characterisation of formulations proceeds by means of PSD-analysis.

An array of at least N different nanoparticle dispersions prepared by the combinatorial method according to the present invention, thus provides, in a preferred embodiment, an average particle size, reported in terms of hydrodynamic radius of dispersed particles in said dispersion formulations, in the range from 10 nm to 10000 nm (10 µm). In a more preferred embodiment dispersed particles in said dispersion formulations have a particle size in the range from 10 to 300 nm and most preferably, dispersed particles in said dispersion formulations are at most 100 nm.

Last but not least, in favour of productivity, according to the present invention a device for the production of m times N different arrays of nanoparticle dispersion formulations is provided, wherein m is an integer and wherein each array is an array as set forth hereinbefore. Multiple times a day this set can be re-used. Multiple arrays are thus envisaged according to the present invention.

More in detail according to the present invention, UV curable ink-jet ink formulations that are particularly suitable for the combinatorial method of making and screening fine, well-dispersed pigment based ink-jet inks comprise following ingredients:
(i) monomers and/or oligomers,
(ii) photo-initiator(s),
(iii) synergistic working additive(s) and
(iv) colourant(s) with, optionally, in addition, one or more surfactant(s), dispersant(s), resin(s), inhibitor(s), hardener(s) and stabilizer(s).

### Monomers and oligomers

UV-curable ink-jet inks contain monomers and/or oligomers, which are polymerised by the curing means of the ink-jet printer. Monomers, oligomers or prepolymers may possess different degrees of functionality, and a mixture including combinations of mono-, di-, tri-and higher functionality monomers, oligomers or prepolymers may be used. These components are curable, typically photo-curable, e.g. UV curable, and should adhere to the ink-receiver surface after printing and serve to bind the colourant.
Adjusting the ratio between the monomers and oligomers is also a method of adjusting the viscosity of the ink. A higher functionality results in a higher viscosity.
Any method of conventional radical polymerization, photo-curing system using photo-acid or photo-base generator, or photo-induced alternating copolymerization may be employed. In general, radical polymerization and cationic polymerization are preferred, and photo-induced alternating copolymerization requiring no initiator may also be employed. Further, a hybrid system of combinations of these systems is also effective.
Cationic polymerization is superior in effectiveness due to lack of inhibition of polymerization by oxygen, however it is slow and its cost is high. If cationic polymerization is used, it is preferred to use an epoxy compound together with an oxetane compound to increase the rate of polymerization.
Radical polymerization is the most widely employed process. Preferred embodiments of radical polymerization will be described below.
Any polymerizable compound commonly known in the art may be employed. Particularly preferred for the ink-jet ink of the ink-jet printing system of the present invention are monofunctional and/or polyfunctional acrylate monomers, oligomers or prepolymers, such as isoamyl acrylate, stearyl acrylate, lauryl acrylate, octyl acrylate, decyl acrylate, isoamylstyl acrylate, isostearyl acrylate, 2-ethylhexyl-diglycol acrylate, 2-hydroxybutyl acrylate, 2-acryloyl-oxyethyl-hexahydrophthalic acid, butoxyethyl acrylate, ethoxydiethylene glycol acrylate, methoxydiethylene glycol acrylate, methoxypolyethylene glycol acrylate, methoxypropylene glycol acrylate, phenoxyethyl acrylate, tetrahydrofurfuryl acrylate, isobornyl acrylate, 2-hydroxyethyl acrylate, 2-hydroxypropyl acrylate, 2-hydroxy-3-phenoxypropyl acrylate, vinyl ether acrylate such as described in US-A 6,310,115, 2-(vinyloxy)ethylacrylate, 2-acryloyloxyethylsuccinic acid, 2-acryloyxyethylphthalic acid, 2-acryloxyethyl-2-hydroxyethyl-phthalic acid, lactone modified flexible acrylate, and t-butylcyclohexyl acrylate, triethylene glycol diacrylate, tetraethylene glycol diacrylate, polyethylene glycol diacrylate,dipropylene glycol diacrylate, tripropylene glycol diacrylate, polypropylene glycol diacrylate, 1,4-butanediol diacrylate, 1,6-hexanediol diacrylate, 1,9-nonanediol diacrylate, neopentyl glycol diacrylate, dimethylol-tricyclodecane diacrylate, bisphenol A EO (ethylene oxide) adduct diacrylate, bisphenol A PO (propylene oxide) adduct diacrylate, hydroxypivalate neopentyl glycol diacrylate, alkoxylated dimethyloltricyclodecane diacrylate and polytetramethylene glycol diacrylate, trimethylolpropane triacrylate, EO modified trimethylolpropane triacrylate, tri (propylene glycol) triacrylate, caprolactone modified trimethylolpropane triacrylate, pentaerythritol triacrylate, pentaerithritol tetraacrylate, pentaerythritolethoxy tetraacrylate, dipentaerythritol hexaacrylate, ditrimethylolpropane tetraacrylate, glycerinpropoxy triacrylate, and caprolactam modified dipentaerythritol hexa-acrylate, or an N-vinylamide such as, N-vinylcaprolactam or N- vinylformamide ; or acrylamide or a substituted acrylamide, such as acryloylmorpholine; and amino functionalised polyetheracrylates such as described in US-A 6,300,388.
Furthermore, methacrylates corresponding to the above-mentioned acrylates may be used with these acrylates. Of the methacrylates, methoxypolyethylene glycol methacrylate, methoxytriethylene glycol methacrylate, 4-(vinyloxy)butylmethacrylate, vinyl acrylates such as described in US-A 6,310,115 (known as its EP-A 0 997 508 counterpart), hydroxyethyl methacrylate, phenoxyethyl methacrylate, cyclohexyl methacrylate, tetraethylene glycol dimethacrylate, and polyethylene glycol dimethacrylate are preferred due to their relatively high sensitivity and improved adhesion to an ink-receiver surface.
Furthermore, the ink-jet inks may also contain polymerizable oligomers. Examples of these polymerizable oligomers include epoxy acrylates, aliphatic urethane acrylates, aromatic urethane acrylates, polyester acrylates, and straight-chained acrylic oligomers.

### Photo-initiators

A catalyst called a photo-initiator typically initiates the polymerization reaction. The photo-initiator requires less energy to activate than the monomers and oligomers to form the polymer.
The photo-initiator absorbs light and is responsible for the production of free radicals or cations. Free radicals or cations are high-energy species that induce polymerization of monomers, oligomers and polymers and with polyfunctional monomers and oligomers thereby also inducing cross-linking.
A preferred amount of initiator is 1-10 weight % of the total ink weight, and more preferably 1-6 weight % of the total ink weight.
Irradiation with actinic radiation may be realised in two steps by changing wavelength or intensity. In such cases it is preferred to use 2 types of initiator together.

Photo-initiators are necessary for free radical curing and may include, but are not limited to, the following compounds or combinations thereof: benzophenone and substituted benzophenones, 1-hydroxycyclohexyl phenyl ketone, thioxanthones such as isopropylthioxanthone,2-hydroxy-2-methyl-1-phenylpropan-1-one, 2-benzyl-2-dimethylamino- (4-morpholinophenyl) butan-1-one, benzil dimethylketal, bis (2,6- dimethylbenzoyl)-2,4,4-trimethylpentylphosphine oxide, 2,4,6-trimethylbenzoyldiphenylphosphine oxide, 2-methyl-1- [4-(methylthio)phenyl]-2-morpholinopropan-1-one, 2,2-dimethoxy-1, 2-diphenylethan-1-one or 5,7-diiodo-3- butoxy-6-fluorone, diphenyliodonium fluoride and triphenylsulfonium hexafluophosphate.
Suitable photo-initiators for ink-jet ink formulations include Irgacure® 184, Irgacure® 500, Irgacure® 907, Irgacure® 369, Irgacure® 1700, Irgacure® 651, Irgacure® 819, Irgacure® 1000, Irgacure® 1300, Irgacure® 1870, Darocure® 1173 and Darocure® 4265 available from CIBA SPECIALTY CHEMICALS, Lucerin TPO available from BASF AG, Esacure® KT046, Esacure® KIP150, Esacure® KT37 and Esacure® EDB available from LAMBERTI, H-Nu® 470 and H-Nu® 470X available from SPECTRA GROUP Ltd. and isopropyl-thioxanthone.

### Inhibitors

A polymerization inhibitor to restrain polymerization by heat or actinic radiation in an ink-jet ink may be added to the formulation. It is preferred to add an inhibitor during preparation of the colourant dispersion. Various compounds are known as polymerization inhibitors, and these compounds may be employed without modification. Examples of polymerization inhibitors include phenol type antioxidants, hindered amine light stabilisers, phosphor type antioxidants, hydroquinonemonomethyl ether commonly used in (metha)acrylate monomers, and hydroquinone, t-butylcatechol, pyrogallol may also be used. Of these, a phenol compound having a double bond in molecules derived from acrylic acid is particularly preferred due to its having a polymerization-restraining effect even when heated in a closed, oxygen-free environment. Suitable inhibitors are, for example, Sumilizer® GA-80, Sumilizer® GM and Sumilizer® GS produced by Sumitomo Chemical Co., Ltd.
Since excessive addition of these polymerization inhibitors will lower the ink sensitivity to curing, it is preferred that the amount capable of preventing polymerization be determined prior to blending. The amount of a polymerization inhibitor is generally between 200 and 20000 ppm of the total ink weight.
Suitable combinations of compounds which decrease oxygen polymerization inhibition with radical polymerization inhibitors are: 2-benzyl-2-dimethylamino-1-(4-morpholinophenyl)-butane-1 and 1-hydroxy-cyclohexyl-phenyl-ketone; 1-hydroxy-cyclohexyl-phenyl-ketone and benzophenone; 2-methyl-1[4-(methylthio)phenyl]-2-morpholinopropane-1-on or 2-methyl-1[4-(methylthio)phenyl]-2-morpholinopropane-1-on and diethyltuioxanthone or isopropylthioxanthone; and benzophenone and acrylate derivatives having a tertiary amino group, and addition of tertiary amines. An amine compound is commonly employed to decrease an oxygen polymerization inhibition or to increase sensitivity. However, when an amine compound is used in combination with a high acid value compound, the storage stability at high temperature tends to be decreased. Therefore, specifically, the use of an amine compound with a high acid value compound in ink-jet printing should be avoided.

Synergistic additives may be used to improve the curing quality and to diminish the influence of the oxygen inhibition. Such additives include, but are not limited to ACTILANE® 800 and ACTILANE® 725 available from AKZO NOBEL, Ebecryl® P115 and Ebecryl® 350 available from UCB CHEMICALS and CD 1012, Craynor CN 386 (amine modified acrylate) and Craynor CN 501(amine modified ethoxylated trimethylolpropane triacrylate) available from CRAY VALLEY.

### Colourants

The ink-jet ink formulations further comprise at least one colourant and any colourant may be used to impart the desired colour to the ink. In particular embodiments of the present invention the colourant may include at least one pigment, one dye, or a combination thereof. A wide variety of organic and inorganic dyes and pigments, alone or in combination may be selected for use in the ink compositions of this invention. The pigment particles should be sufficiently small to permit free flow of the ink through the ink jet printing device, especially at the ejecting nozzles that usually have a diameter ranging from 10 µm to 50 µm. The pigment particle size also has an influence on the pigment dispersion stability, which is critical throughout the life of the ink. It is also desirable to use small particles for maximum colour strength.
Accordingly, the average particle diameter may be from about 0.005 µm to about 15 µm. Preferably, the pigment particle size may range from about 0.005 to about 5 µm, more preferably from about 0.005 to about 1 µm, and most preferably from about 0.005 to about 0.3 µm. Pigment particle sizes outside these ranges may, of course, be used as long as the objectives of the present invention are achieved.
The pigment can be black, cyan, magenta, yellow, red, blue, green, brown, mixtures thereof, and the like. For example, suitable pigment materials include carbon blacks such as Regal 400R, Mogul L, Elftex 320 from Cabot Co., or Carbon Black FW18, Special Black 250, Special Black 350, Special Black 550, Printex 25, Printex 35, Printex 55, Printex 150T from Degussa Co., and Pigment Black 7, 11, 26 and 28. Additional examples of suitable pigments are disclosed in, for example US-A 5,389,133 to Gundlach et al.. Suitable pigments include, for instance, as red pigments Pigment Red 1-10, 12, 19, 22, 23, 31, 38, 41, 43, 48:1, 48:2, 48:3, 48:4, 48:5, 49:1, 49:2, 52:1, 53:1, 57:1, 58:4, 63:1, 81, 81:1, 81:3, 81:4, 81:X, 88, 101, 104, 108, 112, 122, 123, 144, 146, 149, 166, 168, 169, 170, 177, 178, 179, 184, 185, 208, 216, 226, 257; as violet pigments Pigment Violet 1, 3, 19, 23, 29, 30, 37, 50, and 88; as blue or cyan pigments: Pigment Blue 1, 1:2, 1:X, 15, 15:1, 15:2, 15:3, 15:4, 15:6, 16, 17-1, 22, 27, 28, 29, 36, 56, 60, 61 and 62; as green pigments: Pigment Green 7, 26, 36, and 50; as yellow pigments: Pigment Yellow 1, 3, 12, 13, 14, 17, 34, 35, 37, 42, 55, 74, 81, 83, 93, 94, 95, 97, 108, 109, 110, 126, 128, 137, 138, 139, 151, 153, 154, 155, 157, 166, 167, 168, 177, 180, 185 and 193; as white pigments: Pigment White 6, 18, and 21; as orange pigments Pigment Orange 5, 13, 16, 34, as brown pigments Pigment Brown 6, 7, 7:X, Pigment Metal 1, Pigment Metal 2, and bridged aluminum phthalocyanine pigments.
Furtheron the pigment may be chosen from those disclosed in Industrial Organic Pigments, Production, Properties, Applications, second edition, W. Herbst, K. Hunger ; VCH, 1997.
Most preferred pigments are Pigment Yellow 128, 93, 17, 74, 138, 139, 154, 185, 180; Pigment Red 122, 57:1, 184; Pigment Blue 15:3, 15:4, and carbon black.
While carbon black is usually used as the colouring material in black ink, it exhibits a high ultraviolet absorption, resulting in problems of rather low sensitivity in UV curing methods. Therefore, black ink using plural colour pigments other than titanium black or carbon black, may be used to produce black images exhibiting a good UV transparency. Thus, the black ink works quite effectively in ink-jet printing to improve interior hardening in shadow areas where a large amount of ink is specifically ejected in one spot with plural colours. Further, it also works effectively in bi-directional printing. The particles of the pigment dispersed in the ink-jet ink should have a particle size of less than 10 µm, preferably less than 3 µm, and most preferably less than 1 µm. The average particle size of pigment particles is preferably 0.05 to 0.5 µm. By controlling the particle size, clogging of the print head nozzle can be prevented, and ink storage stability, ink transparency and ink curing sensitivity can be maintained.
The pigment may, but need not, be in the form of a dispersion comprising a dispersant also called pigment stabiliser. The latter may be, for example, of the polyester, polyurethane of polyacrylate type, especially in the form of a high molecular weight block copolymer, and would typically be incorporated at 2.5% to 100% by weight of the pigment. Suitable examples are DISPERBYK (ex BYK Chemie) or SOLSPERSE (ex Zeneca) dispersants. A detailed list of non-polymeric as well as some polymeric dispersants appears in, for example, McCutcheon's Functional Materials, North American Edition, Manufacturing Confectioner Publishing Co., Glen Rock, N.J., pp. 110-129 (1990). Other pigment stabilisers are disclosed in DE 19636382, US-A's 5,720,802 and 5,713,993, PCT/GB95/02501, US-A 5,085,689 and GB 2303376. The pigment or dye may be present in the ink composition in any effective amount, generally from about 0.5 to about 20 % by weight of the ink.

### Resins

The ink-jet ink formulations may further contain a resin in order to obtain a stable dispersion of the colourant in the ink-jet ink. Resins are not specifically restricted, but the following resins are preferred: petroleum type resins (e.g., styrene type, acryl type, polyester, polyurethane type, phenol type, butyral type, cellulose type, and rosin) and thermoplastic resins (e.g., vinyl chloride, vinylacetate type). Concrete examples of these resins include acrylate copolymers, styrene-acrylate copolymers, acetalised and incompletely saponified polyvinyl alcohol, and vinylacetate copolymers. Commercial resins are known under the tradenames Solsperse® 32000 and Solsperse® 39000 available from AVECIA, EFKA® 4046 available from EFKA CHEMICALS BV, Disperbyk® 168 available from BYK CHEMIE GMBH.
The resin content in the ink-jet ink is preferably controlled to give a viscosity of less than 100 mPa.s, preferably less than 50 mPa.s, and more preferably less than 30 mPa.s at a shear rate of 100s⁻¹.

### Dispersants and surfactants

Addition of a suitable surfactant or a compound with surface active properties can be used to decrease the dispersive surface energy of the ink-jet ink. A fluorinated or silicone compound may be used as a surfactant, however, a potential drawback is bleed-out after image formation because the surfactant does not cross-link. It is therefore preferred to use a copolymerizable monomer having surface-active effects, for example, silicone-modified acrylates, silicone modified methacrylates, fluorinated acrylates, and fluorinated methacrylates. Specific examples are disclosed in WO 9929787A(XAAR TECHNOLOGY LTD) and WO 9929788 A (XAAR TECHNOLOGY LTD). Suitable examples of surfactants for use in the ink-jet ink formulations include, but are not limited to, ACTILANE® 800 available from AKZO-NOBEL, Tego glide® 410, Tego glide® 435, Tego glide® 440, Tego glide® 450, Tego flow® 300, Tego flow® 425, Tego flow® ZFS460, Tego rad® 2100, Tego rad® 2200-N, Tego rad® 2600, Tego rad® 2700,Tego disperse® 610, Tego disperse® 710, Tego wet® ZFS453 and Tego wet® 250 available from DEGUSSA, Dow corning 67® available from DOW CORNING, Surfadone® 300 available from INTERNATIONAL SPECIALTY PRODUCTS, FC-430®, FC-171® and FC-431® available from 3M, BYK® 306, BYK® 333, BYK® antiterra-u, Disperbyk® 108 and BYK® antiterra 204 available from BYK CHEMIE GMBH, and EFKA® 47 and EFKA® 400 available from EFKA CHEMICALS.

### Organic solvents

It can be advantageous to add an extremely small amount of an organic solvent to the ink-jet ink formulations in order to improve adhesion to the ink-receiver surface after UV curing. In this case, the added solvent can be any amount in the range which does not cause problems of solvent resistance and VOC, and preferably 0.1-5.0%, and particularly preferably 0.1-3.0%. Suitable organic solvents include alcohol, aromatic hydrocarbons, ketones, esters, aliphatic hydrocarbons, higher fatty acids, carbitols, cellosolves, higher fatty acid esters. Suitable alcohols include, methanol, ethanol, propanol and butanol. Suitable aromatic hydrocarbons include toluene, and xylene. Suitable ketones include methyl ethyl ketone and methyl isobutyl ketone.

### Other Additives

Inks of the UV-curable type may include additives such as biocides - as metal or metal oxide (e.g. silver silver oxide, titanum dioxide, zinc oxide) nanoparticles, without however being limitated thereto -, buffering agents, anti-mold agents, pH adjustment agents, electric conductivity adjustment agents, chelating agents, anti-rusting agents, polymerisation inhibitors, light stabilisers, and the like. Such additives may be included in the ink jet inks in any effective amount, as desired. Examples of pH controlling agents suitable for inks of the UV-curable type include, but are not limited to, acids; bases, including hydroxides of alkali metals such as lithium hydroxide, sodium hydroxide and potassium hydroxide. The amount included will depend, of course, on the specific component being included. Furtheron, the ink composition of the UV-curable type may also comprise surfactants and photoinitiator stabilisers. Suitable photoinitiator stabilisers include those disclosed in EP 0 465 039. Suitable surfactants are preferably of the non-ionic type, for example FLUORAD FC430 (ex 3M Corp.). Such surfactants when present are preferably included in an amount of 0.1% to 10% by weight of the total composition. Compositions may further contain organic solvents, such as alcohols, fluorinated solvents and dipolar aprotic solvents. Preferably methanol, ethanol, propanol, 1-butanol, 1-pentanol, 2-butanol, t.-butanol, glycol, glycolethers, N-methylpyrrolidone, N,N-dimethylacetamid, N,N-dimethylformamid, 2,4-pentanedione, and hexafluoroaceton are used.
Ink-jet ink formulations may further contain a dendrimer.
In formulating final ink-jet ink formulations, certain physical properties should be satisfied. For example, ink compositions for use in ink-jet recording processes should have appropriate viscosity and surface tension characteristics. So it is preferred that such a formulation has a viscosity of from about 1 to about 75 mPa.s at 25°C. The surface tension is preferably from 20 to 72 mN/m and most preferably from 20 to 60 mN/m.

Apparatuses for radiation curing in case of curing inks are known to those skilled in the art and are commercially available. For example, the curing proceeds with medium pressure mercury vapour lamps with or without electrodes, or pulsed xenon lamps. These ultraviolet sources usually are equipped with a cooling installation, an installation to remove the produced ozone and optionally a nitrogen inflow to exclude air from the surface of the product to be cured during radiation processing. An intensity of 40 to 240 W/cm in the 200-400 nm region is usually employed. An example of a commercially available ultraviolet medium-pressure electrodeless mercury vapour lamp is the model VPS/1600 curing system of Fusion UV systems Ltd., UK. A pulsed xenon flash lamp is commercially available from IST Strahlentechnik GmbH, Nurtingen, Germany. Using the Fusion model one has also the possibility to use metal halide doped Hg vapour or XeCl excimer lamps, each with its specific UV emission spectrum. This permits a higher degree of freedom in formulating the curing composition: a more efficient curing is possible using the lamp with the most appropriate spectral characteristics.

High energy ionizing radiation such as X-rays, µ-rays, µ-rays, electron beam curing and accelerated electrons may also be used to accomplish curing of an ink composition.

The ink jet receiver materials to which the ink composition of the present invention can be jetted are not limited and include e.g. paper, coated paper, polyolefin coated paper, cardboard, wood, composite boards, plastic, coated plastic, canvas, textile, metal and ceramics.

A particular advantage attained by means of application of the method according to the present invention as set forth is that the formulation design as envisaged is attained much faster and with much smaller amounts of components used.

Moreover parallel screenings on very small amounts are made available, e.g. by making use of a 96-tip probe on microtiterplates.

No intermediate manual steps (such as addition and removal of beads as applied in complex techniques described hereinbefore) are required. The pigment-based dispersion thus obtained is in a stable nanoparticular form, does not crystallise, aggregate, flocculate or precipitate out of said medium within a time of at least one week and, more preferably at least one month.

And last but not least a fully autonomous automated workflow is available, starting at the compositional sheet (followed by automated preparation, automated dispersing, automated screening/measuring) and ending at a data-sheet, provided with a data file.

### Screening

As used herein, the term "screening" is testing, performed on one or more members of a library of materials to determine if a property or compound of interest is present. A screening may be simple and provide only minimal information or data or may be more complex and provide complex information or data. Generally, screening is divided up into levels depending on the type and amount of information that is provided. The first level is a primary screen, which is the fastest or highest throughput screening level. A primary screening, because of the need for fast rates, should be able to screen arrays at a pace that accommodates the method of array preparation. Such a primary screening should thus be able to screen in a day the nanodispersion arrays that are formulated in a day, with the formulation rate being adjusted to accommodate screening and/or vice versa. In the context of this invention, a primary screening will test and determine at least one property or compound in the members of the array as e.g. particle size and particle size distribution. A primary screening will typically be either a parallel visual screen, a parallel optical screen or a high throughput screen as e.g. a viscosity screen and/or a surface tension screen, wherein parallel visual or optical screening is preferred.

Apart from a first screening the next level is secondary screening, wherein it is envisaged to provide more information on array members that pass the primary screen. In some embodiments, secondary screening will require preparation of larger quantities of nanodispersions that are selected from the primary screening and in other embodiments, the secondary screening will be performed on arrays that have been selected from those members of arrays that have passed the primary screening as e.g. an array constructed only of members from previous libraries. Since secondary screening typically is performed on fewer samples than primary screening, the secondary screen may typically take longer than the primary screening and provides additional data. Thus, the secondary screening can be chosen from the same list of screens discussed herein, but may also include a stability test, melting point tests (e.g. such as differential scanning calorimetry), pH testing, chromatography (e.g. size exclusion chromatography), spectroscopy (e.g. UV-VIS absorption), dynamic light scattering for measuring the average particle size, for example FOQELS and FODLS (Fiber Optic Dynamic Light Scattering) or another test to determine material compositions or other tests known to those skilled in the art of nanodispersion technology.

For example, stability screening can be performed by observing the stability of the nanodispersion array member(s) at a first time and possibly a second time, determining any change in nanodispersion stability. Typically, stability is determined by testing the size of the dispersed phase (e.g. by light scattering) or the composition of the nanodispersion (e.g. by chromatography) or by visually observing whether a nanodispersion has formed. The first time will be near to time of nanodispersion formation and may be performed in the range from 2 minutes to 48 hours, less than 24 hours being preferred. Stability at a first time may be a typical primary screening, determining e.g. if a nanodispersion has been formed. A second stability determination may proceed at about 24 hours from the time of nanodispersion formation to as much as 1,2,3 or 4 weeks from the time of formation, thus providing longer term stability testing. Another option is aging of nanodispersions between two or more stability tests. Aging of nanodispersions may be accomplished through thermocycling, shearing, shaking or stirring. Thermocycling is preferred, with the temperature for cycling varying from room temperature to up to 75°C, and preferably at least about 40°C. After primary and secondary screening, focussed arrays selected within compositional regions showing the most desirable results, are prepared. Starting from a library of lots of recipes, preparation conditions, and stabilization characterisation, formulation-processing-property-relationships can be generated by numerical procedures, e.g. fuzzy logic or neuronal networks. This allows a higher level screening in a promising region of the parameter space, e.g. in view of a more detailed investigation, making use of smaller steps in variations of e.g. preparation conditions, as e.g. temperature. This higher level screening loops can be refined, as long the stabilization properties will become better.

### Examples

While the present invention will hereinafter be described in connection with preferred embodiments thereof, it will be understood that it is not intended to limit the invention to those embodiments.

While ultrasonic or ultraturrax techniques were performed, all dispersions were kept at a temperature of 15°C, unless otherwise specified in the description of the examples hereinafter.

In a first experiment ink-jet ink dispersions were prepared by weighing 600 mg of pigment powder on an Autodose Powdernium® MTM solid handler into small glass test tubes having an outer diameter of 13 mm and an height of 75 mm.

The rack with tubes was then moved to an Anachem SK233 liquid handler to add an amount as tabulated in Table 1 of a stock solution of a polymeric dispersant and distilled water. Dosing on the SK233 was done in volumetric mode.

After magnetic stirring on a 60-position Variomag stirrer for 30 minutes, the test tubes were further used in a next dispersion improving step.

**Table 1**

| Ink number | Pigment type | Pigment weight (mg) | Polymer stabilizing dispersant | Polymer volume (µl) | Distilled water (µl) |
|---|---|---|---|---|---|
| I_1 | PB15:3 | 500 | PSPAA1 | 1930 | 70 |
| I_2 | PR122 | 500 | PSPAA1 | 1930 | 70 |
| I_3 | PY74 | 500 | PSPAA1 | 1930 | 70 |
| I_4 | PB15:3 | 600 | PSPAA2 | 390 | 2700 |
| I_5 | PB15:3 | 600 | PSPAA2 | 1950 | 1500 |
| PB = Pigment Blue; PR = Pigment Red; PY = Pigment Yellow | | | | | |

PSPAA1 and PSPAA2 are polystyrene-polyacrylic acid polymeric dispersants, in stock solution concentrations of 22 wt% and 44 wt% respectively.

### Examples 1-6: COMParatives 1-5; INVentive Examples 1-6:

In a first experiment said set of inks was measured without dispersion improvement treatment and after an improvement treatment on a Tomtec Autogizer equiped with individual Sonics® probes of maximum 130 Watt and a 6 mm diameter probe tip. The device was operated in a fast serial way, with as main variation the duration of the ultrasonic treatment. After the dispersion improvement treatment the resulting dispersion was diluted by a factor of about 5000 (depending on the extinction coefficient of the colourant). This was done on the SK233 liquid handler in 2 or 3 separate steps in order to obtain a high dilution accuracy (e.g. 700 µl of the MK8490 in 34.3 ml distilled water, and then again 400 µl of this diluted dispersion into 34.6 ml of distilled water). An absorption spectrum of this diluted dispersion was recorded on a Shimadzu 3401PC UV-VIS NIR spectrophotometer and the absorption intensity was measured at 615 nm and 830 nm. The ratio of these absorption values I615/I830 was tabulated in Table 2 as the "Dispersion Quality Factor" (DQF), "COMP" referring to COMParatives (COMP) 1-5, "INV EX" referring to INVentive Examples 1-6.

It is clear from these experiments set forth in Table 2 hereinafter that without a dispersion improvement step the dispersion quality factor is lower than 5, indicating that a lot of large pigment clusters/agglomerates remain present in the ink, and, as a result, the jetting behaviour in real ink jet printing heads will not be good. By introducing a fast serial ultrasonic dispersing aid the dispersion quality factor can be increased. With a low concentration of polymeric dispersant only moderate improvements are realised. With higher concentrations of said polymeric dispersant better dispersion quality factors can be realised. If compared with the traditional and time consuming method to develop and test inks, our methodology provides excellent discrimination between good and bad compositions on a mg-scale of pigments used.

**Table 2**

| Example | Ink | Dispersion Treatment | DQF |
|---|---|---|---|
| COMP 1 | I_1 | None | 3.8 |
| COMP 2 | I_2 | None | 2.3 |
| COMP 3 | I_3 | None | 2.9 |
| COMP 4 | I_4 | None | 2.3 |
| COMP 5 | I_5 | None | 3.1 |
| INV EX 1 | I_4 | 20 min. US on TOMTEC | 5.1 |
| INV EX 2 | I_4 | 60 min. US on TOMTEC | 6.0 |
| INV EX 3 | I_4 | 120 min. US on TOMTEC | 7.2 |
| INV EX 4 | I_5 | 20 min. US on TOMTEC | 10.2 |
| INV EX 5 | I_5 | 60 min. US on TOMTEC | 16.3 |
| INV EX 6 | I_5 | 120 min. US on TOMTEC | 21.6 |

### Examples 7-9:

The same ink as in example 1 was used, but instead of an ultrasonic treatment on the Tomtec Autogizer, the robot was equiped with high speed homogenisers (Ultraturrax, high speed cutters) and the inks of the examples were treated as described in Table 3. After the dispersion improvement treatment the dispersion quality factors were determined as described for example 1.

**Table 3**

| Example | Ink | Dispersion Treatment | DQF |
|---|---|---|---|
| COMP 1 | I_1 | None | 3.8 |
| INV EX 7 | I_1 | 9 min. UT at 60% on TOMTEC | 11 |
| INV EX 8 | I_1 | 50 min. UT at 30% on TOMTEC | 10 |
| INV EX 9 | I_1 | 30 min. UT at 60% on TOMTEC | 12 |

It is clear that the dispersion improvement step, based upon a rapid parallel/serial high speed cutting principle yields better dispersions than without a dispersion improvement step, while small amounts of chemicals have been used and an efficient workflow can be followed.

### Comparative example 6 and examples 10-15

In this experiment a UV curing ink (I_6) was prepared by weighing 90 mg of Mitsubishi Carbon Black MA8 pigment on the Autodose Powdernium MTM, and adding on the SK233 liquid handler 360 mg of a 20% stock solution of Solsperse 32000 and 2550 mg of monomer DPGDA to end up with a predispersion of 3% pigment and 2.4% Solsperse in the monomer.

All of the dispersions were prepared in small test tubes and stirred for 30 minutes on a multi-position magnetic stirrer. Then the different test tubes were placed in a rack that was cooled with ice water and that was placed in an Sonics® 24-Multi-tip parallel ultrasonic processor having a maximum power of 750 Watt. The output power was set to 40% and the different tubes were agitated during a period of time as indicated in Table 4. After the dispersion improvement treatment, a screening was done for particle size distribution. The mean particle size is given in Table 4 as the quality factor for the obtained dispersion.

It is evident from these data of the Table 4 hereinafter that the parallel ultrasonic treatment leads to smaller mean pigment particle sizes, well below 100 nm, thus being very useful for high quality ink jet inks.

**Table 4**

| Example | Ink | Dispersion Treatment | Particle size (nm) |
|---|---|---|---|
| COMP 6 | I_6 | None | 250* |
| INV EX 10 | I_6 | 1 hour on 24-probe SONICS US | 88 |
| INV EX 11 | I_6 | 2 hour on 24-probe SONICS US | 87 |
| INV EX 12 | I_6 | 3 hour on 24-probe SONICS US | 81 |
| INV EX 13 | I_6 | 4 hour on 24-probe SONICS US | 83 |
| INV EX 14 | I_6 | 5 hour on 24-probe SONICS US | 71 |
| INV EX 15 | I_6 | 6 hour on 24-probe SONICS US | 67 |

| | | | |
|---|---|---|---|
| * mean particle size, with even a minor number of more than 1 µm! | | | |

### Examples 16-18:

The inks (I_1 - I_3) were prepared as described in examples 1-3 and used on the Sonics® 24-probe ultrasonic excitation in order to improve the typical dispersion quality. The parallel ultrasonic treatment was performed for these examples during 20 hours at 40% of the maximum power of the Sonics® processor. After this dispersion improvement process the dispersion quality factors were determined as described in example 1. The results are given in Table 5 hereinafter. As illustrated therein these examples show that by using a very small amount of chemicals it is not only possible to make a clear distinction between different pigment/dispersant formulations, but that it is even possible to bring the parallel screening process to a quality level compatible with typical high quality "fabrication quality" dispersions (traditionally prepared on large scale and making use of a slow process).

**Table 5**

| Example | Ink | Dispersion Treatment | DQF |
|---|---|---|---|
| COMP 1 | I_1 | None | 3.8 |
| COMP 2 | I_2 | None | 2.3 |
| COMP 3 | I_3 | None | 2.9 |
| INV EX 16 | I_1 | 20 hours US on 24-pins SONICS | 49 |
| INV EX 17 | I_2 | 20 hours US on 24-pins SONICS | 26 |
| INV EX 18 | I_3 | 20 hours US on 24-pins SONICS | 28 |

### Examples 19-24:

The inks (I_1 - I_3) were prepared as described in examples 1-3, but now on an even smaller scale. A 96-well microtiter plate of 1 ml vials was used on both the Autodose Powdernium NTM solid handler and the SK233 liquid handler to make 400 µl dispersions of inks I_1, I_2, and I_3. These disperions were used on the Sonics® 24-probe ultrasonic excitation in order to improve the typical dispersion quality.

The parallel ultrasonic treatment was done for these examples during 4 or 20 hours at 30% of the maximum power of the Sonics® processor, the temperature of the dispersions was kept at 20°C using a cooling circuit.

After this dispersion improvement process the dispersion quality factors were determined as described in example 1.

The results are given in Table 6. As illustrated in these examples, summarised hereinafter, it is shown again that even by making use of a very small amount of chemicals it is not only possible to make a clear distinction between different pigment/dispersant formulations, but that it is even possible to bring the parallel screening process to a quality level compatible with typical high quality fabrication quality dispersions (that are traditionally prepared on large scale, making use of a slow process).

**Table 6**

| Example | Ink | Dispersion Treatment | DQF |
|---|---|---|---|
| COMP 1 | I_1 | None | 3.8 |
| COMP 2 | I_2 | None | 2.3 |
| COMP 3 | I_3 | None | 2.9 |
| INV EX 19 | I_1 | 4 hours US on 24-pins SONICS | 34 |
| INV EX 20 | I_2 | 4 hours US on 24-pins SONICS | 23 |
| INV EX 21 | I_3 | 4 hours US on 24-pins SONICS | 33 |
| INV EX 22 | I_1 | 20 hours US on 24-pins SONICS | 74 |
| INV EX 23 | I_2 | 20 hours US on 24-pins SONICS | 51 |
| INV EX 24 | I_3 | 20 hours US on 24-pins SONICS | 45 |

### Examples 25-33

The same experiment as described for examples 22-24 was done, but now the temperature of the cooling bath for the microtiter plate was changed as indicated in Table 7. The microtiter plate was covered with a lid in order to prevent the dispersion from evaporating and concentrating during the ultrasonic treatment.
The results of the resultant disperion quality are also tabulated in Table 7.

**Table 7**

| Example | Ink | Dispersion Treatment | DQF |
|---|---|---|---|
| COMP 1 | I_1 | None | 3.8 |
| COMP 2 | I_2 | None | 2.3 |
| COMP 3 | I_3 | None | 2.9 |
| INV EX 25 | I_1 | 20 hours US at 20 degrees C | 74 |
| INV EX 26 | I_1 | 20 hours US at 15 degrees C | 94 |
| INV EX 27 | I_1 | 20 hours US at 8 degrees C | 97 |
| INV EX 28 | I_2 | 20 hours US at 20 degrees C | 51 |
| INV EX 29 | I_2 | 20 hours US at 15 degrees C | 63 |
| INV EX 30 | I_2 | 20 hours US at 8 degrees C | 63 |
| INV EX 31 | I_3 | 20 hours US at 20 degrees C | 45 |
| INV EX 32 | I_3 | 20 hours US at 15 degrees C | 60 |
| INV EX 33 | I_3 | 20 hours US at 8 degrees C | 64 |

It is clear that the dispersion improvement process is better if the temperature of the dispersion is kept low. Higher temperatures not only lead to possible problems of evaporation and fluctuations in the concentration, but the dispersing process itself seems to be better at lower temperatures. With respect to said temperature as a characteristic parameter, temperature reduction with at least 5°C is advantageously applied during the step of improving the dispersion quality.

### Examples 34-42:

The same experiments as described for examples 7-9 were performed except that in the test tubes used on the Tomtec Autogizer additional milling beads were added and tested in this fast serial /parallel methodology.

Different milling beads have been tested, the typical properties of which are tabulated in Table 8.

**Table 8**

| Bead number | Chemical class | Mean particle diameter (µm) |
|---|---|---|
| B_1 | Glass | 60 |
| B_2 | Glass | 230 |
| B_3 | Glass | 40 |
| B_4 | Glass | 35 |
| B_5 | Glass | 20 |
| B_6 | LD - PE | 180 |
| B_7 | PS-DVB | 100 |
| B_8 | PS-DVB | 50 |
| B_9 | Hybrid glass | 12 |
| LD-PE = Low density Poly Ethylene; | | |
| PS-DVB = polystyrene-divinyl benzene; | | |
| Hybrid glass = TOSPEARL from TOSHIBA, Tokyo, Japan. | | |

After an ultraturrax dispersion improvement step the dispersion quality factor was determined as described in example 1. The results are tabulated in Table 9.

The results given hereinafter indicate that a fast serial/parallel methodology to improve the dispersion quality can be performed on a small sample size scale, while the addition of extra particles in the sample tubes can slightly improve the process, or reduce the treatment time. If compared with the ultrasonic dispersion improvement process the ultraturrax (high speed cutter) technology does not yield improvements as efficient as attained with ultrasonic technology.

**Table 9**

| Example | Ink | Beads | Dispersion Treatment | DQF |
|---|---|---|---|---|
| COMP 1 | I_1 | No | None | 3.8 |
| COMP 2 | I_2 | No | None | 2.3 |
| COMP 3 | I_3 | No | None | 2.9 |
| INV EX 34 | I_1 | No | 60 minutes UT on TOMTEC | 16 |
| INV EX 35 | I_2 | No | 60 minutes UT on TOMTEC | 9 |
| INV EX 36 | I_3 | No | 60 minutes UT on TOMTEC | 11 |
| INV EX 37 | I_1 | 100 mg B_1 | 15 minutes UT on TOMTEC | 17 |
| INV EX 38 | I_2 | 100 mg B_1 | 15 minutes UT on TOMTEC | 8 |
| INV EX 39 | I_3 | 100 mg B_1 | 15 minutes UT on TOMTEC | 11 |
| INV EX 40 | I_1 | 100 mg B_2 | 15 minutes UT on TOMTEC | 14 |
| INV EX 41 | I_2 | 100 mg B_2 | 15 minutes UT on TOMTEC | 6 |
| INV EX 42 | I_3 | 100 mg B_2 | 15 minutes UT on TOMTEC | 7 |

### Examples 43-66:

The same experiment as described in example 20 was performed, but now a microtiter plate with a volume of 2 ml was used and the final volume was increased to 800 µl. Milling beads were added to the final prepared pre-dispersion and then the full microtiter plate was set on the Sonics® 24-pins ultrasonic processor at 30% of the full power for dispersion quality improvement.

The dispersion quality factor was determined as described in example 1. The results are tabulated in Table 10. In the experimental design set up some experiments have been repeated twice or even three times, not only in order to fill each site of the plate, but also in order to control the reproducibility.

**Table 10**

| Example | Ink | Beads type | Beads (mg) | Dispersion Treatment | DQF |
|---|---|---|---|---|---|
| COMP 2 | I_2 | No | 0 | None | 2.3 |
| INV EX 43 | I_2 | B_3 | 50 | 4 hours on SONICS-24 | 30 |
| INV EX 44 | I_2 | B_4 | 100 | 4 hours on SONICS-24 | 73 |
| INV EX 45 | I_2 | None | 0 | 4 hours on SONICS-24 | 20 |
| INV EX 46 | I_2 | B_4 | 300 | 4 hours on SONICS-24 | 421 |
| INV EX 47 | I_2 | B_3 | 400 | 4 hours on SONICS-24 | 911 |
| INV EX 48 | I_2 | B_3 | 75 | 4 hours on SONICS-24 | 57 |
| INV EX 49 | I_2 | None | 0 | 4 hours on SONICS-24 | 17 |
| INV EX 50 | I_2 | B_5 | 150 | 4 hours on SONICS-24 | 42 |
| INV EX 51 | I_2 | B_3 | 300 | 4 hours on SONICS-24 | 564 |
| INV EX 52 | I_2 | B_4 | 300 | 4 hours on SONICS-24 | 392 |
| INV EX 53 | I_2 | B_5 | 300 | 4 hours on SONICS-24 | 45 |
| INV EX 54 | I_2 | B_5 | 75 | 4 hours on SONICS-24 | 35 |
| INV EX 55 | I_2 | B_5 | 75 | 4 hours on SONICS-24 | 36 |
| INV EX 56 | I_2 | B_4 | 150 | 4 hours on SONICS-24 | 118 |
| INV EX 57 | I_2 | B_3 | 150 | 4 hours on SONICS-24 | 97 |
| INV EX 58 | I_2 | B_4 | 75 | 4 hours on SONICS-24 | 53 |
| INV EX 59 | I_2 | None | 0 | 4 hours on SONICS-24 | 18 |
| INV EX 60 | I_2 | B_4 | 150 | 4 hours on SONICS-24 | 122 |
| INV EX 61 | I_2 | B_4 | 75 | 4 hours on SONICS-24 | 57 |
| INV EX 62 | I_2 | B_5 | 50 | 4 hours on SONICS-24 | 32 |
| INV EX 63 | I_2 | B_4 | 300 | 4 hours on SONICS-24 | 430 |
| INV EX 64 | I_2 | B_5 | 300 | 4 hours on SONICS-24 | 45 |
| INV EX 65 | I_2 | B_5 | 400 | 4 hours on SONICS-24 | 64 |
| INV EX 66 | I_2 | B_5 | 150 | 4 hours on SONICS-24 | 44 |

From the results in Table 10 it is clear that remarkable and unexpected improvements can be obtained by adding dispersing beads to the dispersions to be treated in a fast serial, fast serial/parallel and fast parallel dispersion improvement process.
A step of adding beads to the dispersion is advantageously included and is highly recommended. More preferably, adding beads to the dispersion comprising a pigment is performed in a bead weight amount being larger than pigment weight amount, as, e.g., more than 50 mg for a weight of 50 mg of a pigment, and even more preferably in an amount of more than twice the said pigment weight amount.
It is also visible from these data that excellent dispersions can be obtained, clearly attaining the typical quality one can reach in a standard fabrication process. The methodology as described in the present invention is clearly providing an excellent tool in order to improve ink jet dispersions. By making use of only small amounts of chemicals, unambiguous conclusions with respect to optimal ink design can be drawn very fast.

### Examples 67-76

Five additional experiments as described for examples 43-66 were repeated, except that a full experimental design was done with as variations: bead type (B_1 - B_9), bead concentration (0, 75, 150, 300 mg), vial volume (400, 800, 1200 µl).

The pigment and polymeric stabiliser were kept constant as in I_2. The ultrasonic treatment was done at 30% of the full power on the Sonics® -24 ultrasonic processor for a fixed period of 4 hours. At the end of this procedure the dilutions were done on 3 levels directly in a standard microtiterplate.

The final dilution in the microtiterplate layout was measured on a custom made optical microplate reader having both reflection and transmission measuring capabilities. In this case the full spectra of the 24 diluted samples were recorded via a fiber optic coupled oceanOptics® spectral analysis, the registering probe being fixed but the microtiter plate, coupled on an XY-stage performing the full spectral analysis in a fast serial way. In this way a full plate could be analysed in less than 1 minute.

The measured intensity at peak absorbance was related to the measured intensity at the bathochromic edge of the absorption spectrum again, and the dispersion quality factor (DQF) was determined as explained in example 1.

The results of the full experimental analysis are given in Table 11, wherein the tabulated values are representing the statistical average values of more than 15 individual experiments.

Fig. 4 is referred to as it shows a photograph of a custom made microtiter plate reader, offering the ability to determine a full visual spectrum in a fast serial way via a fiber-optic coupled oceanOptics® spectrum analyser, connected to a moving head on a XY-stage and connected to the spectrum analyser via an optical fiber, wherein said device provides both transmission and reflection measuring capabilities.

From these experiments, the results of which have been summarised hereinafter, it becomes clear that the nature of the particles added into the dispersion improvement step has a big influence on the final quality. Glass beads perform better than PS or PE beads, smaller particle diameters perform better than larger ones. However, there is also a limit on the particle size: if it is dropping below a diameter of 20 µm, then the improvements in the dispersion process are very small again. Beads added in the dispersion improvement step are advantageously composed of glass. It is even more preferred that the beads have a particle size diameter in the range from 25 µm to 200 µm.

**Table 11**

| Example | Ink | Beads type | Beads particle diameter (µm) | Dispersion Treatment | DQF |
|---|---|---|---|---|---|
| COMP 2 | I_2 | No | - | None | 2.3 |
| INV EX 67 | I_2 | No | - | 4 hours on SONICS-24 | 27 |
| INV EX 68 | I_2 | B_1 | 60 | 4 hours on SONICS-24 | 82 |
| INV EX 69 | I_2 | B_2 | 230 | 4 hours on SONICS-24 | 38 |
| INV EX 70 | I_2 | B_3 | 40 | 4 hours on SONICS-24 | 179 |
| INV EX 71 | I_2 | B_4 | 35 | 4 hours on SONICS-24 | 319 |
| INV EX 72 | I_2 | B_5 | 20 | 4 hours on SONICS-24 | 103 |
| INV EX 73 | I_2 | B_6 | 180 | 4 hours on SONICS-24 | 58 |
| INV EX 74 | I_2 | B_7 | 100 | 4 hours on SONICS-24 | 81 |
| INV EX 75 | I_2 | B_8 | 50 | 4 hours on SONICS-24 | 33 |
| INV EX 76 | I_2 | B_9 | 12 | 4 hours on SONICS-24 | 17 |

### Experiments 77-85:

In microtiterplates having a vial size of 2 mm, ink composition I_2 was prepared as described in examples 43-66, but the amount of pigment was lowered to 80 mg per vial. To these final ink preparations bead B_3 were added in 2 different concentrations.

Then the ultrasonic dispersion improvement step was performed on the Sonics® -24 parallel ultrasonic probe, but the ultrasonic agitation time was reduced from 60 to 30 and even to 15 minutes only. At the end the samples were diluted as described in the previous examples and the DQF was determined as described earlier.

The results are summarised in Table 12 hereinafter.

**Table 12**

| Example | Ink | Beads | Beads concentration (mg) | Dispersion Treatment Time (30% Sonics® -24) | DQF |
|---|---|---|---|---|---|
| COMP 2 | I_2 | No | - | None | 2.3 |
| INV EX 77 | I_2 | No | - | 15 | 7.0 |
| INV EX 78 | I_2 | No | - | 30 | 8.5 |
| INV EX 79 | I_2 | No | - | 60 | 9.5 |
| INV EX 80 | I_2 | B_3 | 300 | 15 | 14 |
| INV EX 81 | I_2 | B_3 | 300 | 30 | 21 |
| INV EX 82 | I_2 | B_3 | 300 | 60 | 48 |
| INV EX 83 | I_2 | B_3 | 600 | 15 | 15 |
| INV EX 84 | I_2 | B_3 | 600 | 30 | 22 |
| INV EX 85 | I_2 | B_3 | 600 | 60 | 88 |

These data indicate that parallel dispersion improvement process and also a fast serial/parallel dispersion improvement process can speed up the development of ink jet dispersions to a considerable extent.

As an ultimate test to prove the usefulness of the dispersion improvement processes described above, the comparative formulations COMP 7-9 having inks I_1, I_2 and I_3 respectively from Table 7, were treated in an ultrasonic bath as such during 1 hour, thus not with an individual, dedicated energy addition step by means of an N-pins ultrasonic probe.
DQF-values were determined and summarised in Table 13.

**Table 13**

| Example | Ink | Dispersion Treatment | DQF |
|---|---|---|---|
| COMP 7 | I_1 | 1 hour ultrasonic bath | 4.2 |
| COMP 8 | I_2 | 1 hour ultrasonic bath | 2.3 |
| COMP 9 | I_3 | 1 hour ultrasonic bath | 3.6 |

If compared with the DQF values in Table 7, no better (versus the 2.3 for I_2) or slightly higher values (versus the 3.8 figure for I_1 and the 2.9 figure for I_3) than comparative figures can be obtained when applying an ultrasonic bath treatment during 1 hour. It is clear that an improvement of the dispersion quality factor up to a level of at least 5 cannot be attained yet!

By making use of very small amounts of chemicals and incorporating a parallel (or fast serial/parallel) dispersion improvement step assisted by the addition of dispersion improvement beads, and coupling this preparation and dispersion step to a fully automated screening step, a very powerfull technique is obtained. Advantageously said method is performed with a sample volume of less than 3 ml for each of said formulations.

Characterisation of formulations advantageously proceeds by directly screening said formulations in a microtiter plate by means of spectral analysis. Instead of making use of a spectroscopic technique to determine the DQF, another alternative of a fast serial screening process was also tested. The diluted ink samples were spotted (using the SK233 liquid handler) in screenplates, commercially available from Schleicher & Schuell, Germany. Screenplates GF50®, GF53®, and GF55® were used. Small microvials from the PL-PSDA® PSD-device of Polymer Labs, UK, were placed under these screenplates, and then 1.5 ml of ink was dispensed into the screen plates (from Schleicher & Schuell GmbH, Dassel, Germany) using the SK233 liquid handler. It took about 3 minutes for the 1.5 ml of ink to be filtered into the measuring vial on the GF50 screenplate, while this time was reduced to 1.5 minutes for GF53, but took about 12 minutes for screenplate GF55. After all small vials were filled, they were placed in the caroussel of the automated particle size measurement device of Polymer Labs, UK, and the evaluation of the particle size distribution started automatically and autonomously (eventually overnight). At the end of the screening process all curves on the particle size distribution of all inks were available.

In another embodiment, characterisation of formulations advantageously proceeds by means of PSD-analysis. This method was very suitable in order to identify the best ink sets that could be fabricated. For a rough screening process, the fast optical screening methodology was already offering satisfying results. Fig. 5 is referred to as it shows an example of a PSD measurement as performed on the automated PL-PSDA device, wherein the particle size distribution for a single ink is depicted. All samples were available in a caroussel so that fast serial measurements could proceed autonomously.

The present invention thus provides useful methods for an integrated combinatorial approach towards the discovery of novel and/or the preparation of ink-jet ink nanodispersions. Herein it is envisaged that the approach creates a nanodispersion of aqueous, solvent-based and radiation curable ink-jet inks, wherein the desired dispersion is selected as providing the desired properties in a most suitable way. The work-flow provides identification of the most suitable ink-jet ink dispersion and one or more combinatorial libraries of such ink-jet ink nanodispersions are designed.

The present invention thus provides greatly improved workflow for parallel preparation, homogenizing and screening of formulations such as ink-jet inks. It should be understood that the above description is intended to be illustrative and not restrictive.

Many embodiments and variations of the invention will become apparent to those skilled in the art upon review of this disclosure. The scope of the invention should, therefore, be determined not with reference to the above description, but instead should be determined with reference to the appended claims along with the full scope of equivalents to which such claims are entitled.

## Claims

1. A combinatorial method of making and testing an array of nanoparticle formulations comprising the steps of
- making multi-compositional formulations;
- improving the dispersion quality of these formulations by high speed parallel homogenizing;
- rapid serial, semi-parallel or parallel **characterising** said formulations,
**characterised in that** said step of high speed parallel homogenizing proceeds by providing energy to said dispersion via an array of tips.

2. Method according to claim 1, wherein said energy is ultrasonic energy.

3. Method according to claim 2, wherein in said step of high speed parallel homogenizing an N-tip probe is connected to an ultrasonic source, N being an integer.

4. Method according to claim 1, wherein said energy is high shear mixing energy.

5. Method according to claim 4, wherein said high shear mixing energy is provided via a high speed mixer with a rotation speed of more than 10,000 r.p.m..

6. Method according to claim 3, wherein said N-tip probe is provided with N tips, arranged in a linear or in a two-dimensional arrangement.

7. Method according to claim 6, wherein said N-tip probe is provided with N tips, arranged in a linear or in a two-dimensional arrangement, in order to make M nanoparticle formulations, wherein N and M are integers.

8. Method according to claim 7, wherein N and M are related by the formula 4 < N < M.

9. Method according to claim 7, wherein N and M are related by the formula M/N = n, wherein n is an integer.

10. Method according to any one of the claims 1 to 9, wherein said formulations are differing in at least one characteristic parameter, wherein said parameter is selected from the group consisting of composition, temperature and pH-value.

11. Method according to any one of the claims 1 to 10, wherein the step of characterisation proceeds by screening of the improved formulations and is performed by techniques selected from the group consisting of optical, electrical, magnetic, chemical, biochemical and physicochemical techniques.

12. Method according to any one of the claims 1 to 10, wherein the step of characterisation proceeds by screening of the improved formulations and is performed by parallel measurement techniques selected from the group consisting of optical, electrical, magnetic, chemical, biochemical and physicochemical techniques.

13. Method according to claim 11 or 12, wherein the step of characterisation proceeds by screening of the improved formulations and is performed by an optical method selected from the group consisting of visual inspection of nanoparticle dispersion formulations, optical transmission at selected wavelength(s) and quasi-elastic light scattering.

14. Method according to any one of the claims 10 to 13, wherein, with respect to said temperature as a characteristic parameter, temperature reduction with at least 5°C is applied during the step of improving the dispersion quality.

15. Method according to any one of the claims 1 to 14, wherein a step of adding beads to the dispersion is included.

16. Method according to claim 15, wherein adding beads to the dispersion comprising a pigment is performed in a bead weight amount being larger than pigment weight amount.

17. Method according to claim 15, wherein adding beads is performed in an amount of more than twice the said pigment weight amount.

18. Method according to any one of the claims 15 to 17, wherein said beads are composed of glass.

19. Method according to claim 18, wherein said beads have a particle size diameter in the range from 25 µm to 200 µm.

20. Method according to any one of the claims 1 to 19, wherein said method is performed with a sample volume of less than 10 ml for each of said formulations.

21. Method according to any one of the claims 1 to 20, wherein said characterisation of formulations proceeds by directly screening of said formulations in a microtiter plate by means of spectral analysis.

22. Method according to any one of the claims 1 to 20, wherein said characterisation of formulations proceeds by means of PSD-analysis.

23. An array of at least N different nanoparticle dispersions prepared by the combinatorial method according to any one of the claims 1 to 22, wherein an average particle size, reported in terms of hydrodynamic radius, of dispersed particles in said dispersion formulations is from 10 nm to 10000 nm.

24. Device for the production of m times N different arrays of nanoparticle dispersion formulations, wherein m is an integer and wherein each array is an array according to claim 23.
